# EUROPEAN PATENT APPLICATION

(11) **EP 3 332 754 A1**
(43) Date of publication of application: **13.06.2018**
(21) Application number: 17203956.2
(22) Date of filing: 28.11.2017
(51) Int. Cl.: A61F 5/058

(54) **MEDICAL SUPPORT**

(30) Priority: 28.11.2016 SE 1651557
(71) Applicant: Danderyds sjukhus AB, 182 88 Danderyd (SE)
(72) Inventor: Burnic, Selmin, 170 62 Solna (SE)
(74) Representative: AWA Sweden AB

(57) **Abstract**

A medical support (100), comprising a first portion (200) comprising a groove (210) in the first portion extending longitudinally along the length of the first portion and configured to at partially enclose the lower part of the leg. The medical support further comprises a second portion (300) comprising a base portion (310) extending longitudinally along the length of the second portion, and at least one support structure (320) arranged at at least one longitudinal side of the base portion and configured to support at least a portion of the upper part of the leg. The first portion and the second portion are interconnected by a length-adjustable element (400) arranged in the longitudinal direction of the medical support and configured to adjust the length of the medical support.

## Description

### FIELD OF THE INVENTION

The present invention generally relates to the field of medical devices. More specifically, the present invention relates to a medical support for a patient suffering from a broken, dislocated and/or fractured hip.

### BACKGROUND OF THE INVENTION

A broken or fractured hip is a fracture in the upper portion of the thighbone, or femur. Most hip fractures happen to elderly people, and the fractures are usually caused by falls. It should be noted that with age, the bones may naturally lose some strength and are thereby more likely to break, even from a minor fall.

According to studies, approximately 1.6 million hip fractures occur worldwide each year, and by 2050, this number could reach between 4.5 million and 6.3 million. Furthermore, it should be noted that the lifetime risk of having a hip fracture may be as high as 1 in 6. Another serious issue related to accidents of this kind is the time patients will have to wait in hospitals before being operated. As it is not uncommon that a patient may have to wait more than 24 hours, or even more, for an operation, this wait for a patient suffering from a hip fracture may be unpleasant, distressful and/or painful.

Hence, it will first be appreciated that the number of people that suffer a hip fracture is ever-increasing, and that queues at hospitals for getting the adequate treatment or operation may be relatively long. Furthermore, considering that hip fractures are invariably associated with chronic pain, reduced mobility, disability and/or an increasing degree of dependence, and that a broken hip may be a relatively serious condition for a person at any age and almost always requires surgery, there is an increasing need for providing an adequate medical support for the afflicted persons. Today, when patients with a hip fracture are taken to hospital and are arranged in hospital beds, the medical staff may often try to support the patient's leg and/or hip by towels, cushions, or the like. These may not only be inadequate and/or awkward for the actual support of the patient, but their use may also be non-hygienic.

In addition, there may also be a need for providing a medical support of the above-mentioned kind which may be adjusted to fit persons and/or patients having different physical properties. For example, it may be desirable to provide a medical support which may fit a child and/or an adolescent as well as an adult.

US3762405A shows an orthopedic traction leg splint assembly. The assembly is comprised of a forward L-shaped trough-like shell to support the calf, ankle and foot of a patient. A rear trough-like shell supports the patient's thigh. Both shells may be lined with a thick, resilient pad removably secured by an adhesive. The shells are joined by pairs of hinges including pivotally connected slotted hinge bars, which may be adjusted to space the shells and position them angularly.

However, it should be noted that the construction of the splint assembly shown in US3762405A is relatively complex and circumstantial. Consequently, the splint assembly is relatively difficult to adjust by the patient and/or medical staff. A patient and/or medical staff trying to adjust the assembly may hereby run the risk of injuring himself/herself upon the adjustment operation itself. Furthermore, the relatively complex and circumstantial construction may lead to an inadequate or incorrect adjustment of the splint assembly, which consequently may lead to an incorrect or even detrimental splint assembly when used by a patient. Moreover, an incorrectly adjusted assembly may be painful for a patient using said assembly.

Hence, alternative solutions are of interest, which are able to efficiently and conveniently support a patient suffering from a hip fracture, especially when the patient is lying down, e.g. on a hospital bed.

### SUMMARY OF THE INVENTION

It is an object of the present invention to mitigate the above problems and to provide a medical device, arranged for supporting a patient suffering from a fractured, broken and/or dislocated hip, which medical device is conveniently attachable to a patient and/or easily disinfected.

This and other objects are achieved by providing a medical support having the features in the independent claim. Preferred embodiments are defined in the dependent claims.

Hence, according to a first aspect of the present invention, there is provided a medical support, comprising a first portion arranged to support a lower part of a leg of a patient. The first portion comprises a groove in the first portion extending longitudinally along the length of the first portion and configured to at partially enclose the lower part of the leg. The medical support further comprises a second portion configured to support an upper part of a leg of a patient. The second portion comprises a base portion extending longitudinally along the length of the second portion, and at least one support structure arranged at at least one longitudinal side of the base portion and configured to support at least a portion of the upper part of the leg. The first portion and the second portion are interconnected by a length-adjustable element arranged in the longitudinal direction of the medical support and configured to adjust the length of the medical support.

Thus, the present invention is based on the idea of providing a medical support which primarily is intended for a patient suffering from a fractured, broken and/or dislocated hip. The medical support comprises first and second portions, arranged to support a lower and an upper part of the patient's leg. Moreover, the second portion of the medical support, via the base portion and the support structure, provides a support and/or fixture of the upper part of the patient's leg. The first and second portions are interconnected by a length-adjustable element arranged in the longitudinal direction of the medical support and configured to adjust the length of the medical support. Hence, the medical support provides a medical device which is conveniently attachable and/or adjustable to a patient, and whereby the first and second portions and the element of the medical support, by their respective features, synergetically contribute to an improved support and/or comfort compared to devices according to the prior art.

The present invention is advantageous in that the medical support, apart from its superior support, is relatively stable. Hence, the medical support may ensure a reliable stability for a patient to which the medical support is attached. This is highly advantageous not only regarding healing purposes but also regarding the comfort of the patient, mitigating involuntary movements of the patient's leg which may be painful.

The present invention is further advantageous in that the medical support, via the length-adjustable element, may be easily and conveniently adjusted in length to fit persons/patients having different physical properties.

The present invention is further advantageous in that the medical support is relatively inexpensive to manufacture. Hence, the medical support may be bought by e.g. hospitals, institutes and/or private persons to a relatively low cost. Consequently, the medical support may furthermore be discarded after use and/or if damaged without any significant financial loss.

The present invention is further advantageous in that the medical support may be cleaned and/or disinfected in a relatively easy manner. It will be appreciated that the hole of the first portion may even further contribute to the facilitated cleaning and/or disinfection of the medical support, especially when compared to an arrangement only comprising a cavity for the heel (i.e. no through hole).

The present invention is further advantageous in that the medical support, due to its stability and/or comfort, may lead to the patient feeling calmed and/or assured. This is especially important when considering that hip fractures may be traumatic for many patients.

The present invention is further advantageous in that the medical support, due to its design and/or a somewhat resilient material, may fit persons having different physical properties. The medical support may fit both men and women and/or people of different weight, length, proportions, etc.

The present invention is further advantageous in that the medical support may prevent so called "foot drop", in which the dropping of the forefoot happens due to weakness, irritation or damage to the common fibular nerve or paralysis of the muscles in the anterior portion of the lower leg.

The medical support comprises a first portion arranged to support a lower part of a leg of a patient, the first portion comprising a groove in the first portion extending longitudinally along the length of the first portion and configured to at partially enclose, support and/or fix the lower part of the patient's leg. By the term "groove", it is here meant an (longitudinal) opening, or the like, configured to at least partially accommodate at least a portion of a patient's lower leg. The medical support further comprises a second portion configured to support an upper part of a leg of a patient, the second portion comprising a base portion extending longitudinally along the length of the second portion. Hence, the first portion and the second portion are aligned and arranged along a longitudinal axis of the medical support. Furthermore, at least one support structure arranged is at least one longitudinal side of the base portion and configured to support at least a portion of the upper part of the leg. By "support structure", it is here meant substantially any structure, element, support, or the like, configured to support at least a portion of the upper part of the patient's leg. The first portion and the second portion of the medical support are interconnected by a length-adjustable element arranged in the longtitudinal direction of the medical support and configured to adjust the length of the medical support. By the term "length-adjustable element", it is here meant substantially any element which is extendable and/or retractable into the interior and/or on the exterior of the first and/or second portion of the medical support. Hence, the interconnection between the first portion and the second portion may extend and/or retract the overall length of the medical support. The present embodiment is advantageous in that the medical support may be adjusted to fit persons having different physical properties. For example, the length-adjustable element may be retracted such that the medical support may conveniently fit a relatively short person. Analogously, the medical support may be arranged to fit a relatively long person by extending the length-adjustable element.

According to an embodiment of the present invention, the length-adjustable element comprises a bar comprising a length indicator and a fastening device for securing a desired length of said element. By the term "length indicator", it is here meant substantially any construction, device, or the like, which is configured to indicate the relative or absolute distance (e.g. in centimeters and/or inches) between the first portion and the second portion of the medical support. The present embodiment is advantageous in that the bar comprising the length indicator conveniently and clearly indicates the relative or absolute distance between the first portion and the second portion of the medical support for a patient and/or medical staff. The present embodiment is further advantageous in that the fastening device of the bar may conveniently secure any desired length of the element, and consequently, also of the overall length of the medical support.

According to an embodiment of the present invention, the first portion comprises, at an end portion of the first portion, a cavity in the first portion configured to accommodate a foot of the patient, wherein the cavity at its bottom comprises a hole through the first portion to accommodate the heel of said foot. By the term "cavity", it is here meant a hollow, or the like, wherein the material of the first portion at least partially surrounds and/or encloses the patient's foot. At the bottom of the cavity, there is provided a hole through the first portion to accommodate the heel of the foot. By the term "hole", it is here meant a hole which may have a diameter larger than the diameter of the patient's heel, such that the heel of the patient's foot may project into the hole. The present embodiment is advantageous in that the cavity for the accommodating the patient's foot and the hole for accommodating the patient's heel even further contributes to the support and/or fixture of the lower part of the patient's leg.

According to an embodiment of the present invention, the at least one support structure comprises two support elements arranged at either longitudinal side of the base portion. The support elements project perpendicularly with respect to the longitudinal direction of the medical support and are configured to at least partially enclose at least a portion of the upper part of the leg. Hence, in case medical support is attached to a patient, the two support elements are configured to project on either side of a patient's upper leg. It will be appreciated that in a case the medical support is attached to a patient lying substantially horizontally, e.g. in a hospital bed, the two support elements are arranged to project in a substantially vertical direction. The present embodiment is advantageous in that the two support elements provide an even further support and/or fixation of a patient's leg, as they may mitigate any sideways movement of the patient's leg. Furthermore, the present embodiment is advantageous in that the two support elements provide an even more comfortable medical support.

According to an embodiment of the present invention, the first portion and the second portion are formed as one piece. In other words, the first and second portions of the medical support are formed as integrally manufactured to constitute a one-piece medical support.

According to an embodiment of the present invention, the first portion and the second portion are formed separately. In other words, the first and second portions of the medical support are formed as separate, albeit interconnected units. The present embodiment is advantageous in that a medical support formed in separate pieces may be more easily stored and/or produced. Furthermore, in case of damage of one of the portions, this portion may be discarded and replaced. Hence, the present embodiment may provide a more cost-efficient device compared to devices found in the prior art.

According to an embodiment of the present invention, the medical support may further comprise a strap arrangement configured to be arranged at least partially over the second portion upon attachment of the strap arrangement. The present embodiment is advantageous in that the strap arrangement may conveniently tighten the second portion around the upper portion of the patient's leg.

According to an embodiment of the present invention, the strap arrangement is attachable to the support elements and configured to bias the support elements when the strap arrangement is arranged over the second portion. Hence, the strap arrangement may be configured to bias the two projecting support elements upon attachment such that the support elements become inclined. The present embodiment is advantageous in that the strap arrangement hereby provides a comfortable, stable and/or reliable support of the medical support when provided to a patient's leg.

According to an embodiment of the present invention, the strap arrangement comprises at least one Velcro tape.

According to an embodiment of the present invention, the base portion of the second portion is wedge-shaped and is inclined in a direction from the second portion to the first portion of the medical support. Hence, when the medical support is applied to a patient, the second portion of the medical support, which hereby may be provided under an upper part and/or buttocks of the patient, may be relatively flat. The part of the second portion facing the first portion may, on the other hand, be relatively thick. The present embodiment is advantageous in that the base portion of the second portion of the medical support hereby provides comfort and/or stability for the patient.

According to an embodiment of the present invention, the medical support may be made of a foam material. The present embodiment is advantageous in that the medical support is relatively inexpensive to produce. Furthermore, the present embodiment is advantageous in that the medical support is of relatively light weight.

According to an embodiment of the present invention, the medical support further comprises at least one handle arranged on an underside of the medical support. The present embodiment is advantageous in that the medical support may be relatively easy to pull when applied to a patient lying on a (hospital) bed.

According to an embodiment of the present invention, the underside of the medical support is (relatively) flat. The present embodiment is advantageous in that the medical support may hereby be even more stable.

Further objectives of, features of, and advantages with, the present invention will become apparent when studying the following detailed disclosure, the drawings and the appended claims. Those skilled in the art will realize that different features of the present invention can be combined to create embodiments other than those described in the following.

### BRIEF DESCRIPTION OF THE DRAWINGS

This and other aspects of the present invention will now be described in more detail, with reference to the appended drawings showing embodiment(s) of the invention.
Figs. 1-2 are schematic views of a medical support according to an exemplifying embodiment of the present invention, and
Fig. 3 is a schematic view of a medical support according to an exemplifying embodiment of the present invention provided to a patient.

### DETAILED DESCRIPTION

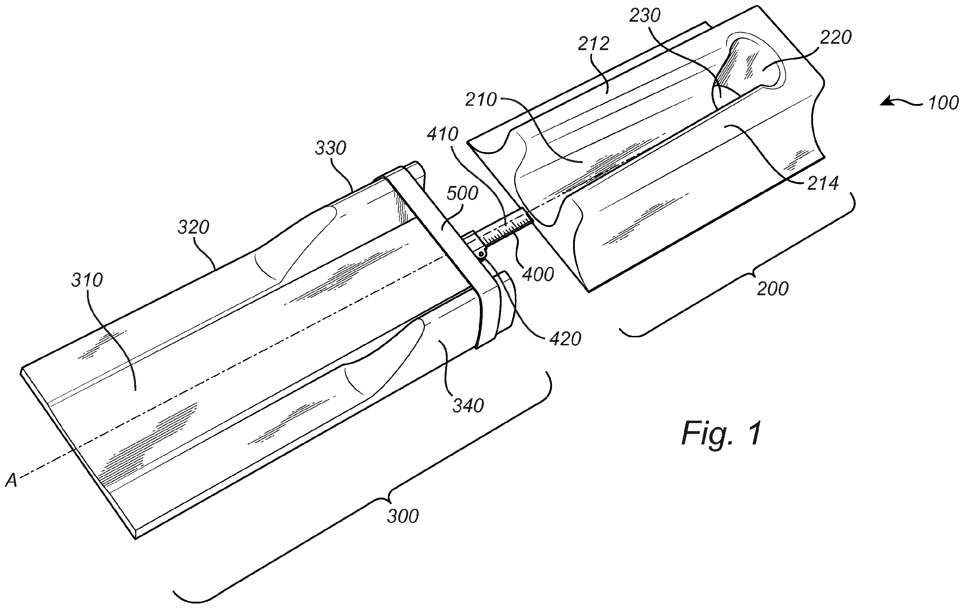

Fig. 1 is a schematic view of a medical support 100 according to an embodiment of the present invention. The medical support 100 comprises a first portion 200 arranged to support a lower part of a leg of a patient, and a second portion 300 arranged to support an upper part of the patient's leg. The first 200 and second portions 300 of the medical support 100 are arranged along a longitudinal axis A of the medical support 100. The first portion 200 comprises a groove 210 extending longitudinally along the length of the first portion 200, i.e. along the axis A. The groove 210, which is at least partially defined on either side by wall portions 212, 214, is configured to at partially enclose the lower part of the patient's leg. A cavity 220 is arranged in the first portion 200 and at an end portion of the first portion 200. The cavity 220 is configured to accommodate a foot of the patient. At the bottom of the cavity 220, there is provided a hole 230 through the first portion 200. The hole 230 is configured to accommodate the heel of the patient's foot.

The second portion 300 of the medical support 100 comprises a base portion 310 extending longitudinally along the length of the second portion 100. The second portion 300 further comprises a support structure 320 arranged on the base portion 310. In Fig. 1, the support structure 320 is exemplified as comprising two support elements 330, 340 arranged at either longitudinal side of the base portion 310. The support elements 330, 340 project perpendicularly with respect to the longitudinal direction of the medical support 100. Hence, when the medical support 100 is arranged horizontally, as shown in Fig. 1, the support elements 330, 340 are configured to project vertically. The support elements 330, 340 are configured to at least partially enclose at least a portion of the upper part of the patient's leg.

In Fig. 1, the first portion 200 and the second portion 300 of the medical support 100 are formed separately. However, it should be noted that the medical support 100 alternatively may be formed integrally into one piece. An end part of the first portion 200 is connected to an end part of the second portion 300 by an element 400 which is adjustable in length. Here, the element 400 is exemplified as a bar 410 comprising a scale/length indicator, and a fastening device 420 for securing a desired length of the element 400. Hence, by shortening or extending the element 400, the total length of the medical support 100 can be adjusted. For example, the medical support 100 can be adjusted by the element 400 to fit a relatively short patient, e.g. a child or an adolescent. Alternatively, the medical support 100 can be adjusted by the element 400 to fit a relatively tall patient, e.g. an adult. It will be appreciated that other devices and/or elements for adjusting the overall length of the medical support 100 may be used.

The medical support 100 further comprises a strap arrangement 500. Here, the strap arrangement 500 is exemplified as comprising a strap which is attached to a first support element 330. The strap is furthermore arranged over the second portion 300 and is releasably attached to the second support element 340, e.g. via a Velcro tape. The strap arrangement 500 is hereby configured to be arranged over an upper part of a leg of a patient.

The base portion 310 of the second portion 300 of the medical support 100 is wedge-shaped. In other words, it is inclined along the longitudinal axis A of the medical support 100 in a direction from the second portion 300 to the first portion 200.

It will be appreciated that the medical support 100 may be made of a foam material.

Although not shown in Fig. 1, there may be provided one or more handles arranged on the first portion 200 and/or the second portion 300 of the medical support 100.

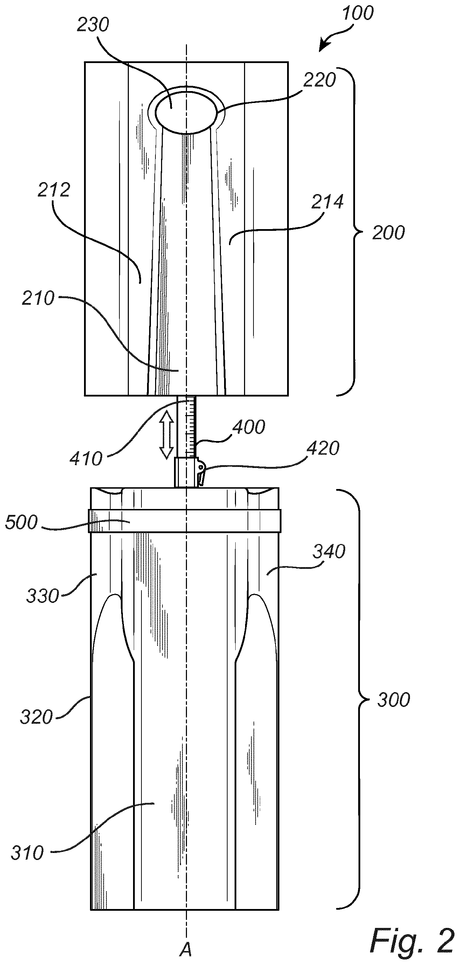

Fig. 2 is a schematic top view of a medical support 100 according to the embodiment of the present invention disclosed in Fig. 1. It will be appreciated that the cavity 220 in the first portion 200 of the medical support 100 comprises a hole 230 through the first portion 200. Furthermore, it should be noted that the base (i.e. underside) of the medical support 100 is relatively large, implying a relatively stable medical support 100.

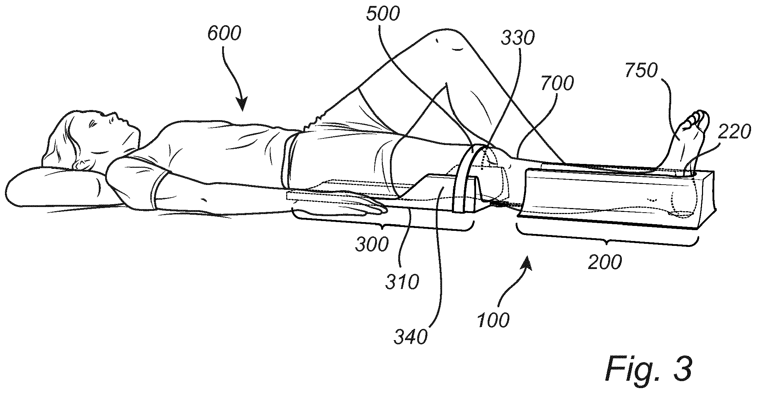

Fig. 3 is a schematic view of a medical support 100 according to an embodiment of the present invention, wherein the medical support 100 is provided to patient 600 lying on a hospital bed. The leg 700 of the patient 600 is arranged in the medical support 100, or, in other words, the medical support 100 is applied and/or provided to the patient's leg. The first portion 200 of the medical support 100 is arranged to support a lower part of the leg 700 of the patient 600, i.e. a part of the leg 700 under the knee. A major portion of the lower part of the leg 700 of the patient 600 is arranged in the groove provided in the first portion 200. Furthermore, the foot 750 of the patient 600 is arranged in the cavity 220 in the first portion 200 of the medical support 100. The heel of the foot 750 of the patient 600 is arranged in a hole at the bottom of the cavity 220.

The second portion 300 of the medical support 100 is arranged to support an upper part of the leg 700 of the patient 600, i.e. above the knee. The base portion 310 of the second portion 300 extends longitudinally under the thigh of the patient 600. The two support elements 330, 340, which are arranged at either longitudinal side of the base portion 310, are configured to support at least a portion of the upper part of the leg 700 of the patient 600.

The person skilled in the art realizes that the present invention by no means is limited to the preferred embodiments described above. On the contrary, many modifications and variations are possible within the scope of the appended claims. For example, it will be appreciated that the figures are merely schematic views of a medical support according to embodiments of the present invention. Hence, any feature and/or element of the medical support 100, such as the first portion 200, the second portion 300 and/or the length adjustable element 400, may have different dimensions, shapes and/or sizes than those depicted and/or described. For example, any portion and/or element of the medical support 100 may be larger or smaller than what is exemplified in the figures.

## Claims

1. A medical support (100), comprising
a first portion (200) arranged to support a lower part of a leg of a patient, said first portion comprising
a groove (210) in said first portion extending longitudinally along the length of said first portion and configured to at partially enclose the lower part of the leg,
wherein said medical support further comprises
a second portion (300) configured to support an upper part of a leg of a patient, said second portion comprising
a base portion (310) extending longitudinally along the length of said second portion, and
at least one support structure (320) arranged at at least one longitudinal side of said base portion and configured to support at least a portion of the upper part of the leg,
wherein said first portion and said second portion are interconnected by a length-adjustable element (400) arranged in the longitudinal direction of said medical support and configured to adjust the length of said medical support.

2. The medical support according to claim 1, wherein the length-adjustable element comprises a bar (410) comprising a length indicator and a fastening device (420) for securing a desired length of said element.

3. The medical support according to claim 1 or 2, wherein the first portion comprises, at an end portion of said first portion,
a cavity (220) in said first portion configured to accommodate a foot of said patient, wherein said cavity at its bottom comprises a hole (230) through said first portion to accommodate the heel of said foot.

4. The medical support according to any one of the preceding claims, wherein said at least one support structure comprises two support elements (330, 340) arranged at either longitudinal side of said base portion, wherein said support elements project perpendicularly with respect to the longitudinal direction of said medical support and are configured to at least partially enclose at least a portion of the upper part of the leg.

5. The medical support according to any one of the preceding claims, wherein said first portion and said second portion are formed as one piece.

6. The medical support according to any one of claims 1-4, wherein said first portion and said second portion are formed separately.

7. The medical support according to any one of the preceding claims, further comprising a strap arrangement (500) configured to be arranged at least partially over said second portion upon attachment of said strap arrangement.

8. The medical support according to claims 4 and 7, wherein said strap arrangement is attachable to said support elements and configured to bias said support elements when said strap arrangement is arranged over said second portion.

9. The medical support according to claim 7 or 8, wherein said strap arrangement comprises at least one Velcro tape.

10. The medical support according to any one of the preceding claims, wherein said base portion of said second portion is wedge-shaped and is inclined in a direction from said second portion to said first portion of said medical support.

11. The medical support according to any one of the preceding claims, wherein said medical support is made of a foam material.

12. The medical support according to any one of the preceding claims, further comprising at least one handle arranged on an underside of said medical support.

13. The medical support according to any one of the preceding claims, wherein the underside of said medical support is flat.
